# EUROPEAN PATENT APPLICATION

(11) **EP 3 866 173 A1**
(43) Date of publication of application: **18.08.2021**
(21) Application number: 21165738.2
(22) Date of filing: 15.03.2013
(51) Int. Cl.: G16H 20/17

(54) **PATIENT CARE DEVICE-SPECIFIC CONFIGURATION OUTPUT**

(30) Priority: 23.01.2013 US 201361755873 P
(62) Divisional of application: 13716522.1
(71) Applicant: Baxter Corporation Englewood, Englewood, CO 80112 (US)
(72) Inventor: SCHNEIDER, Dennis, I., Nashua, NH New Hampshire 03062 (US); TRIBBLE, Dennis, A., Ormond Beach, FL Florida 32174-1808 (US)
(74) Representative: Müller-Boré & Partner Patentanwälte PartG mbB

(57) **Abstract**

A method and system for generation of a patient care device-specific configuration output are provided. The method comprises by an order entry interface, receiving an order that includes at least a portion of a therapy description corresponding to a therapy to be administered to a patient. The method further comprises by a configuration output generator, identifying a patient care device to be used to administer the therapy to the patient, wherein the patient care device has at least one predetermined configuration protocol for therapy administration. The method further comprises by the configuration output generator, analyzing the therapy description in relation to the predetermined configuration protocol to determine if the therapy description conforms to the predetermined configuration protocol. The method further comprises by the configuration output generator, generating a configuration output for the order comprising patient care device configuration data for use in manual configuring of the patient care device for administration of the therapy, the configuration output being based at least in part on at least a portion of each of the corresponding therapy description and the predetermined configuration protocol.

## Description

### RELATED APPLICATIONS

This application claims priority to U.S. Provisional Patent Application No. 61/755,873 filed January 23, 2013, entitled "PATIENT CARE DEVICE-SPECIFIC CONFIGURATION OUTPUT," which application is incorporated herein by reference in its entirety.

### BACKGROUND

Information conveyance errors continue to represent a diverse and widespread challenge in the administration of medical therapies to patients. Specifically, errors may occur when a caregiver is required to configure a patient care device based on instructions associated with a therapy to be administered to a patient (e.g., when a caregiver must transcribe configuration input from one location to the patient care device). In this context, errors may be introduced through a number of modalities including, for example, inaccurate data transcription, ineffective communication modalities, and/or inaccurate data conversion.

One such scenario where errors may be introduced is when the configuration of a patient care device includes the configuration of a configurable infusion device in connection with the administration of an IV fluid corresponding to a dose order generated by a caregiver such as a physician. Given the nature of the therapy involved when administering IV fluids, errors in this context may cause long term injury or even threaten the life of a patient. As such, the reduction of errors associated with the configuration of patient care devices, and in particular configurable infusion devices, continues to be of great importance.

### SUMMARY

The present disclosure is generally directed to generation and/or use of a patient care device-specific configuration output that may be used in connection with the configuration of a patient care device (e.g., a configurable infusion pump or similar apparatus) by a caregiver that is to administer a medication intravenously. Specifically, the configuration output may be based on a therapy administration order and an identified patient care device to be used to administer a therapy. In this regard, upon the identification of the patient care device to be used to administer the therapy, a specific configuration protocol associated with the identified patient care device may be known. As such, when generating the configuration output, the configuration output may correspond to the configuration protocol such as, for example, with respect to the identity of the configuration data needed to configure the patient care device, an order in which the configuration data is presented, or a form in which the configuration data is presented.

Accordingly, it may be appreciated that such a configuration output may be beneficial when configuring a patient care device in a number of respects. For example, by generating the configuration output based on an order and an identified patient care device to be used to administer a therapy, the configuration output may assist an administrating caregiver in correctly configuring the patient care device (e.g., when transcribing information from the configuration output to the patient-care device). In a first respect, generation of the configuration output may include analyzing the order to determine if information corresponding to the configuration of the patient care device has been provided. If not, mechanisms may be employed to receive such information prior to generation of the configuration output. This may help to prevent a situation where an administrating caregiver, upon attempting to configure the patient care device, discovers that configuration data needed to configure the patient care device is missing or the data is conflicting. Advantageously, the method provides for an improved man machine interaction, since obtainment of necessary data for patient care device configuration may be facilitated, e.g., in a centralized manner. Therefore configuration mistakes and/or incompatibilities can be reduced or even avoided. That is, correct configuration data may be provided, thus preventing a situation where an administrating caregiver errs in configuring the patient care device by incorrectly entering data not present in the order (e.g., using data that the administrating caregiver guesses, using data from memory, or otherwise unilaterally attempting to overcome the lack of information). In other words, according to this application, the user, such as an administrating caregiver, is relieved from the mental task of memorizing necessary configuration data or may be relieved from the mental task of learning, memorizing, or even understanding one or more predetermined configuration protocols.

Also, the configuration output as described herein may assist an administrating caregiver by analyzing information from an order to facilitate that the information appearing thereon conforms to the configuration protocol of the identified patient care device. This may include providing data corresponding to the information of the therapy order in the configuration output in a manner such that the data is arranged in conformance with a data input sequence of the configuration protocol. In this regard, the arrangement of data may conform to the data input sequence such that the data corresponding to each subsequent step in the data input sequence is presented sequentially in the configuration output. In this regard, a machine created configuration output may be provided, namely a configuration output that conforms to a data input sequence of a particular patient care device. Hence, according to this application the reliability of the machine based method is improved. Further still, the information may be analyzed to determine that the order information corresponds in form to the configuration protocol. For example, if the units of measure of a value appearing in the order are different than the units of measure of the value to be used in the configuration protocol, the information from the order may be converted to an appropriate form (e.g., the correct units of measure) prior to generation of the configuration output. In this regard, it may be appreciated that the configuration output described herein may assist a caregiver administering a therapy in configuration of a patient care device. Advantageously, the caregiver may be relieved from the mental task of converting different formats to match each other, in particular to one or more configuration protocols. Hence, incorrect or erroneous use of the patient care device may be avoided.

Accordingly, a first aspect includes a method for generation of a patient care device-specific configuration output for a therapy order that corresponds with a therapy to be administered to a patient. The method includes receiving an order that includes a therapy description corresponding to a therapy to be administered to a patient and identifying a patient care device to be used to administer the therapy to the patient. The patient care device has at least one predetermined configuration protocol for therapy administration. The method also includes generating a configuration output for the order, for use in configuring the patient care device for administration of the therapy, based at least in part on at least a portion of each of the corresponding therapy description and the predetermined configuration protocol.

A number of feature refinements and additional features are applicable to the first aspect. These feature refinements and additional features may be used individually or in any combination also with respect to features of the second aspect described below. As such, each of the following features that will be discussed may be, but are not required to be, used with any other feature or combination of features of the first aspect.

For example, in an embodiment, the therapy may comprise administration of an IV fluid to a patient using an infusion device (e.g., a configurable infusion pump). Furthermore, the order may comprise a dose order including a dose description corresponding to the IV fluid to be administered. In this regard, the configuration output may be used to configure the infusion device for administration of the IV fluid to the patient using the infusion device (e.g., by way of an administrating caregiver transcribing data from the configuration output to the infusion device). Generally, "configurable" as used herein may include the ability to receive inputs of one or more operation parameters from a human user (e.g., inputs that are manually input by the human user) that relate to controlling operation of the patient care device for administration of a therapy. Hence, the subject of this application provides an improved and more reliable configuration of e.g. the patient care device, such as an infusion device. The IV fluid may comprise at least one of the following:
a compounded IV admixture;
a premixed IV solution (e.g., a premixed solution of Dextrose 5% in Water with Dopamine);
a customer-prepared non-patient-specific fluid (e.g., a standard Oxytocin drip); or
any other type of IV fluid without limitation.

In an embodiment, the method may also include generating a label comprising the configuration output and affixing the label to a receptacle associated with the therapy. The predetermined configuration protocol may include a predetermined data input sequence for input of a plurality of patient care device configuration data. In this regard, the configuration output may be presented on the label in accordance with at least a portion the predetermined data input sequence. That is, the configuration data may include a plurality of patient care device configuration data that are presented in the same order as the predetermined data input sequence of the identified patient care device. Hence, according to this embodiment, man machine interaction is improved by providing the label that reduces the risk of mis-matching patient care device configuration data and the predetermined data input sequence.

As such, in an embodiment, the method may also include inputting a corresponding plurality of patient care device configuration data from the configuration output presented on the label to the patient care device in accordance with the predetermined data input sequence. The inputting may include entering the corresponding plurality of patient care device configuration data at the patient care device manually by a user. For example, the inputting may include transcribing the patient care device configuration data from the label to the patient care device (e.g., using a user interface of the patient care device). Furthermore, the method may include administering the therapy with the patient care device at least partially based on the inputting step. This may include operating the patient care device based on the patient care device configuration data that is transcribed to the patient care device. In various embodiments, the plurality of patient care device configuration data may include at least one of the following types of data:
data indicative of a location of the patient within a patient care facility;
data indicative of one of a predetermined plurality of dose types;
data indicative of at least one therapeutically active drug;
data indicative of dose concentrations;
data indicative of an administration rate;
data indicative of an administration amount; or
any other appropriate data that may be used to configure a patient care device.

In an embodiment, the identifying step may include identifying one of a plurality of types of patient care devices to be used to administer the therapy to the patient, wherein each one of the plurality of patient care devices has a different predetermined configuration protocol. The identifying step and/or the generating step may be completed, based at least in part, upon a location of the patient within a patient care facility.

In an embodiment, the method may include analyzing the therapy description in relation to at least a portion of the predetermined configuration protocol to determine if the therapy description conforms to the portion of the configuration protocol. The method may include providing an output alert at a user interface device when the therapy description is in nonconformance with the portion of the configuration protocol and receiving, in response to said output alert, therapy information corresponding to the order that conforms to the portion of the configuration protocol, thus the man machine interaction between the user interface of the patient care device and the user of the patient care device, such as the caregiver is improved. The therapy information may be used in the generating step. For example, the method may further include requiring receipt of the therapy information that conforms to the portion of the configuration protocol prior to completion of the generating step.

In an embodiment, the portion of the configuration protocol may include at least one configuration parameter. In this regard, the nonconformance of the therapy description may include at least one missing configuration parameter. Additionally or alternatively, the nonconformance of the therapy description may include contradictory information related to the at least one configuration parameter. In an embodiment, the method may include converting at least a portion of the therapy description from a first form that does not conform with the portion of the configuration protocol to a second form that does conform to the portion of the configuration protocol. In this regard, the converting may include performing a unit of measure conversion on at least one portion of the dose description. In an embodiment, the portion of the configuration protocol may comprise the entire configuration protocol for the identified patient care device. Thus, according to this application, in an automatic manner, ideally without the need of an interference by a user, such as a care provider, non-conforming or even contradictory data may be corrected to comport with a configuration protocol for a patient care device. Therefore, the user is relieved from the mental task to identify non-conforming or even contradictory data. The user may also be relieved from the task of having to memorize how to correct such non-conforming data or even contradictory data.

In an embodiment, the identifying step may be at least partially determined based on at least a portion of the order. That is, the method may include assigning the order to a patient care device at least partially based on a portion of the order. For example, the portion of the dose order on which the identifying step Is at least partially based comprises at least one of:
an identity of the therapy; and/or
a location of the patient.
That is, the identifying of the patient care device (e.g., a configurable infusion device) may be based upon data indicative of the location of the patient within the patient care facility and data indicative of the at least one drug to be administered. In this embodiment, once these data values are obtained, the patient care device may be identified and the exact configuration protocol may be known for generation of a corresponding configuration output. In this regard, the configuration output may include an identification of the patient care device to be used to administer the therapy to the patient (e.g., the patient care device identity may be printed on a label).

In an embodiment at least one of the identifying step and the generating step may be at least partially completed in a computer-automated manner. In this regard, the method may be executed as hardware, software, or a combination thereof to perform one or more steps of the method automatically by a computing device. In other words, the method may be a computer implemented method. For example, as discussed above, the identifying may include automatically assigning the therapy order to a patient care device to be used to administer the therapy. As such, the identification of the patient care device may be automatically performed by a computing device and the generating of the configuration output may also be automatically performed once the patient care device has been identified. Hence, a user, such as a care provider does not need to manually identify a patient care device. Hence, the risk of a wrong identification may be reduced. Other portions of the method may be performed automatically (e.g., by one or more computing devices) without limitation.

In an embodiment, the method may include communicating the configuration output to the patient care device. Furthermore, the method may include displaying the configuration output at the patient care device. As such, the configuration output may comprise machine-readable data that may be manipulated, communicated, and/or displayed by a computing device).

According to yet another aspect, a computer program product is provided that can be stored on a computer readable medium and/or can be implemented as computer processable data stream, wherein the computer program product comprises computer processable instructions, which instructions when read in the memory of a computer and executed by the computer cause the computer to carry out the method(s) as described in general above, and in more specific examples below.

A second aspect includes a system for generation of a patient care device-specific configuration output for use in configuring a patient care device to administer a therapy to a patient. The system may include an order entry interface operable to receive a therapy order that includes at least a portion of a therapy description corresponding to a therapy to be administered to a patient and a configuration output generator. The configuration output generator may be in operative communication with the order entry interface. Furthermore, the configuration output generator is operable to identify a patient care device to be used to administer the therapy to the patient, wherein the patient care device has at least one predetermined configuration protocol for therapy administration. The system may also include a configuration output that is generated by the configuration output generator and is based at least in part on at least a portion of each of the corresponding therapy description and the predetermined configuration protocol.

A number of feature refinements and additional features are applicable to the second aspect. These feature refinements and additional features may be used individually or in any combination also with features of the first aspect described above. As such, each of the following features that will be discussed may be, but are not required to be, used with any other feature or combination of features of the second aspect.

For example, in an embodiment, the therapy may include administration of an IV fluid to a patient using an infusion device. In this regard, the therapy order may comprise a dose order including a dose description corresponding to the IV fluid to be administered. In various embodiments, the IV fluid may include any one or more of the IV fluids discussed above.

In an embodiment, the order entry interface and/or the configuration output generator may be executed by a processor In operative communication with one or more memories. In this regard, one or more memories may store non-transitory computer-readable data corresponding to the execution of the order entry interface and/or the configuration output generator. It may be understood that the order entry interface may be executed by the same or a different processor than the configuration output generator. In an embodiment, the order entry interface may be executed on a completely separate device from the configuration output generator. Furthermore, a single memory may store non-transitory computer-readable data corresponding to the execution of both the order entry interface and the configuration output generator. Alternatively, separate memories may be used in this regard. In either regard, the one or more memories may be provided remotely from a corresponding processor (e.g., over a network) or locally with respect to a corresponding processor.

In an embodiment, the configuration output may comprise a label. That is, the configuration output may be printed on a label. The label may be applied to a receptacle for use in administration of the therapy. Furthermore, the system may include a patient care device of a corresponding type identified by the configuration output generator. The patient care device may have a user interface for configuration of the patient care device. The user interface may correspond to the configuration protocol of the patient care device. As such, the label may include one or more portions of configuration data corresponding to at least a portion of the configuration protocol. In various embodiments, the one or more portions of configuration data may include at least one of the following types of data:
data indicative of a location of the patient within a patient care facility;
data indicative of one of a predetermined plurality of dose types;
data indicative of at least one drug;
data Indicative of dose concentrations;
data indicative of an administration rate;
data indicative of an administration amount; or
any other appropriate data that may be used to configure a patient care device.

In an embodiment, the configuration protocol of the patient care device may include a predetermined data input sequence. In this regard, a label including the configuration output may include a corresponding plurality of configuration data presented on the label in accordance with the predetermined data input sequence. The user interface of the patient care device may be operable to receive the corresponding plurality of configuration data in the predetermined data input sequence. For example, the corresponding plurality of configuration data may be manually entered to the patient care device by a user (e.g., as transcribed by a human user from the label).

In an embodiment, the patient care device may be a configurable infusion pump. Accordingly, the receptacle may include an IV fluid and the receptacle may be in operative communication with an administration set. The administration set may in turn be operatively engaged by the configurable infusion pump for controlled delivery of the IV fluid to a patient. As such, the IV fluid may be administered to the patient at least partially based on the corresponding plurality of configuration data input at the configurable infusion pump.

### BRIEF DESCRIPTION OF THE FIGURES

Fig. 1 is a schematic representation of an embodiment of a work flow for administration of an ordered therapy to a patient using a patient care device.
Fig. 2 represents an embodiment of a configurable patient care device comprising a configurable infusion device that is in operative engagement with an IV fluid contained in a receptacle for administration to a patient.
Fig. 3 depicts a flow chart corresponding to an embodiment of a method for generation of a patient care device-specific configuration output for an order.
Fig. 4 represents an embodiment of a dose order corresponding to an IV fluid to be administered to a patient.
Fig. 5 represents an embodiment of a configuration output corresponding to the dose order of Fig. 4.
Fig. 6 depicts an embodiment of a configuration output comprising a label applied to a receptacle for an IV fluid to be administered to a patient.
Figs. 7A-7E depict an embodiment of a user interface of a configurable infusion device in plurality of different states corresponding a configuration protocol for configuring the infusion device.

### DETAILED DESCRIPTION

The following description is not intended to limit the invention to the forms disclosed herein. Consequently, variations and modifications commensurate with the following teachings, skill and knowledge of the relevant art, are within the scope of the present invention. The embodiments described herein are further intended to explain modes known of practicing the invention and to enable others skilled in the art to utilize the Invention in such, or other embodiments and with various modifications required by the particular applications(s) or use(s) of the present invention.

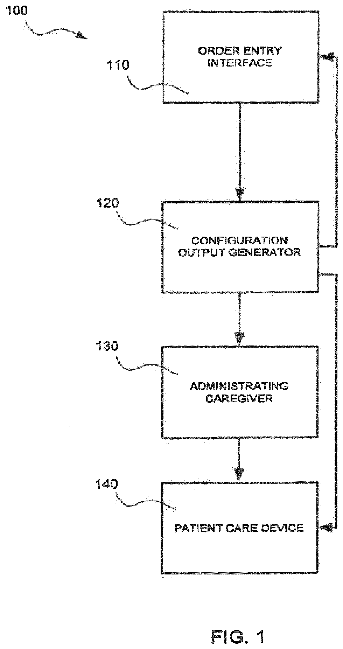

Fig. 1 depicts a schematic representation of an embodiment of a work flow 100 that facilitates generation of a device-specific configuration output for use in configuration of a patient care device 140. Generally, the work flow 100 may include use of an order entry system 110 for receiving an order for a therapy that is to be administered to a patient using the configurable patient care device 140. The order entry system 110 may output data corresponding to an order for receipt by a configuration output generator 120. The configuration output generator 120 may generate a patient care device-specific configuration output that is based on the order and on an identified patient care device. The patient care device specific configuration output may be provided to an administrating caregiver 130. The administrating caregiver 130 may then use the configuration output received from the configuration output generator 120 to configure the patient care device 140 (e.g., that corresponds to the identified patient care device) for administration of the ordered therapy to the patient. Additionally or alternatively, the configuration output generator 120 may provide the configuration output directly to the patient care device 140 (e.g., for display or other use of the configuration output at the patient care device 140). Additionally, as shown, and as will be discussed in greater detail below, the work flow 100 may include a feedback loop between the configuration output generator 120 and the order entry interface 110 that may be used to solicit additional information regarding the order (e.g., once the order is analyzed by the configuration output generator 120).

In an embodiment, the order entry interface 110 may be a computer-based order entry system. That is, the order entry interface 110 may include a graphical user interface (GUI) facilitated by a computing device that allows a caregiver to enter information corresponding to an order for administration to a patient. For example, the computing device may be a networked computing device such as a personal computer, network terminal, mobile device, or other computing device. In this regard, the order entry interface 110 may include at least one processor operable to access a memory storing instructions to control execution of the processor to receive and output an order.

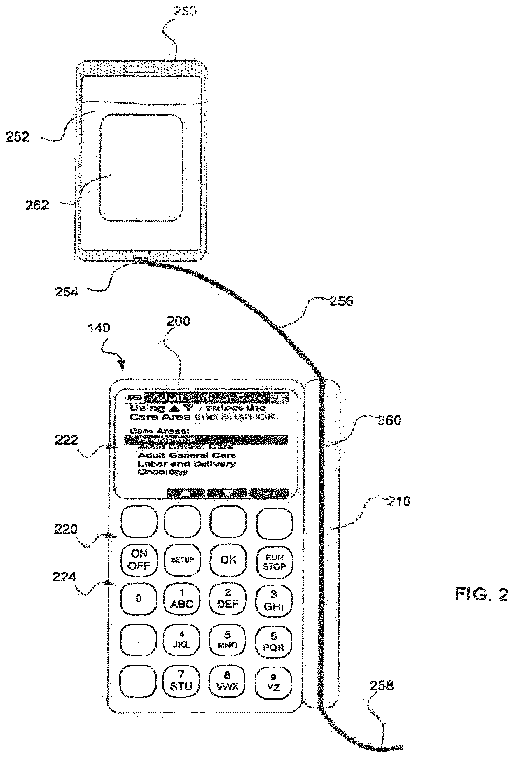

In any regard, the order entry interface 110 may receive information from a caregiver regarding a therapy to be administered to a patient. In an embodiment, the therapy may include the administration of an IV fluid to a patient using a configurable patient care device 140. For example, as depicted in Fig. 2, the configurable patient care device 140 may include an infusion device 200 (e.g., a configurable infusion pump). In this regard, the order received at the order entry interface 110 may be a dose order with dose information corresponding to an IV fluid 252 to be administered to a patient. The IV fluid 252 to be administered to the patent may be contained by a receptacle 250. The receptacle 250 may include an administration port 254 that facilitates connection of an administration set 256 to the receptacle 250. The administration set 256 may be used to facilitate fluid communication between the receptacle 250 and a patient (not shown) at a distal end 258 of the administration set 256.

Additionally, a portion 260 of the administration set 256 may be disposed within a pump portion 210 of the infusion device 200. In this regard, the pump portion 210 may have an appropriate mechanism (e.g., a peristaltic pump device) for control of the flow of the IV fluid 252 through the administration set 256 for administration of the IV fluid 252 to the patient. Other appropriate types of infusion devices 200 may be provided without limitation that allow for control of the administration of an IV fluid 252 to a patient.

In this regard, the infusion device 200 may be configurable to control the pump portion 210 for controlled administration of the IV fluid 252 to the patient. For example, the infusion device 200 may include a graphical user interface (GUI) 220 used to receive configuration instructions from an administering caregiver 130. The GUI 220 may include a display 222 and an input device 224 (e.g., including a plurality of buttons or keys as shown in Fig. 2). Other input devices 224 may be provided including for example, a touch screen input device, a keyboard, or other human-machine interface. In this regard, the administering caregiver 130 may use the input device 224 to input information regarding the ordered administration of the IV fluid 252 to the patient that is in turn reflected on the display 222.

While one example of a patient care device 140 in the form of an infusion device 200 is described above and shown in Fig. 2, it will be appreciated that the disclosure contained herein may extend broadly to any configurable patient care device 140 that may be used in administration of a therapy to a patient. For example, the system 100 may comprise other configurable patient care devices 140 such as patient monitors (e.g., including cardiac monitors, hemodynamic monitors, respiratory monitors, neurological monitors, blood glucose monitors, childbirth monitors, body temperature monitors, etc.), inhalation therapy devices, enteral feeding pumps, respiratory ventilation devices, dialysis devices, or other appropriate configurable patient care devices 140 without limitation.

It is recognized that prior approaches to facilitating the configuration of patient care devices 140 may include risks for errors to occur during the configuration of the patient care device 140. One study by Husch et al. (Insights from the sharp end of intravenous medication errors: implications for infusion pump technology, Qual. Saf. Health Care 2005;14;80-86), the entirely of which is incorporated herein by reference, includes detailed discussion regarding the continued need for error prevention in this context. For example, continuing the example discussed above, it may be that a dose order associated with the administration of the IV fluid 252 is maintained separately from the information regarding the preparation and identification of the IV fluid 252 itself. That is, a receptacle 250 may include a label 262 that bears data regarding the IV fluid 252. However, the order concerning the administration of the IV fluid 252 may be separately maintained from the IV fluid 252 (e.g., in a separate order or medical record system). As such, the administering caregiver 130 may be required to consult multiple sources of data during the configuration of the patient care device 130. This may introduce the potential for errors, for example, in the form of incorrectly sourced data, transcription errors, or other error modalities.

Furthermore, even if the order data is included on the label 262 disposed on the receptacle 250, there may still be incorrect, missing, or misidentified data. For example, the data appearing on the label 262 may require the administering care giver 130 to perform conversions or provide supplemental data to arrive at the necessary input for configuring the patient care device 140. Therefore, in addition to the errors identified above, the potential for errors on the part of the administering caregiver 130 may include mathematical errors, unit of measure conversion errors, or other errors introduced when the administering caregiver 130 attempts to configure the patient care device 140 in accordance with the order.

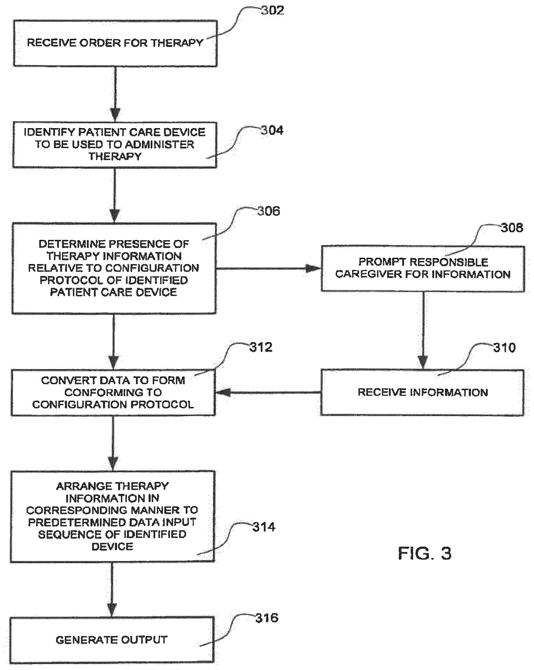

As such, an embodiment of the configuration output generator 120 described herein may be operable to generate a patient care device-specific configuration output that is generated with respect to, and corresponds to, both the therapy to be administered to the patient and the patient care device 140 to be used to administer the therapy. For example, the configuration output generator 120 may generally perform a method 300 as shown in Figure 3. In this regard, the configuration output generator 120 may comprise hardware, software, or combinations thereof. For example, in an embodiment, the configuration output generator 120 may at least comprise a general purpose processor and a memory. As such, instructions corresponding to the method 300 as described below may be provided in the form of non-transitory machine-readable data that is accessible by the processor to control the operation thereof to perform the method 300 described below. As such, the configuration output generator 120 may include or be executed on a computing device such as a networked computing device, a network terminal, a mobile device, or other appropriate computing device. Additionally or alternatively, at least portions of the method 300 may be performed by application specific integrated circuits (ASICs), field gate arrays (FGAs), or other appropriate devices for executing functionality as will be appreciated by one of ordinary skill in the art. Accordingly, any or all steps of the method 300 may be performed in a computer-automated manner.

The method 300 may include receiving 302 and order for a therapy. For example, the order may be received via an order entry interface 110 as referenced in Fig. 1. Once the order is received 302, the method 300 may include identifying 304 the patient care device to be used to administer the ordered therapy to the patient. In this regard, the identifying 304 may include identifying one of a plurality of types of devices that may be used in the administration of the therapy to the patient. Each type of device may include a different predetermined configuration protocol. For example, in the case of a configurable infusion device 200 as described in Fig. 2, a facility may have more than one type of infusion device. Each of the different types of infusion devices 200 may have a predetermined configuration protocol that differs with respect to each other type of infusion device 200. In this regard, the identifying may include, rather than identifying a specific one of patient care devices 140 of a facility to be used to administer a therapy, a type of patient care device 140 may be determined such that the predetermined configuration protocol is determined for the device to be used in the administration of the ordered therapy.

In another embodiment described in greater detail below, the identifying step 304 may include selecting or assigning a patient care device at least partially based on the order. For example, in the context of a dose order, the assigning of an appropriate infusion device 200 may be based upon the type of infusion, the identity of the drug to be infused, the patient to whom the IV fluid is to be administered, the current location of the patient, or any other factor. In an embodiment, the identifying 304 is based on the current location of the patient and the identity of the drug to be infused. Once this information is known, an appropriate infusion device 200 may be selected such that the configuration protocol thereof is known. In any regard, once the predetermined configuration protocol for a patient care device 140 to be used to administer the therapy has been identified 304, then information corresponding to the order may be analyzed and/or processed to output a configuration output based on at least a portion of the information regarding the therapy order and the identification of the patient care device.

For example, as shown in Fig. 3, an embodiment of the method 300 may include determining 306 the presence of information in the therapy order that corresponds to the configuration protocol of the identified patient care device 140. In a particular scenario, it may be determined that a portion of the information required in the configuration of the patient care device 140 is not provided In the therapy order. Furthermore, a scenario may exist where the therapy order includes conflicting information regarding the administration of the therapy. Other scenarios may be provided for where there is some deficiency in the information provided in the order. In any regard, the method 300 may include prompting 308 a responsible caregiver for information. This may include alerting a responsible caregiver of the issue (e.g., via the order entry interface 110) and may include prompting the responsible caregiver for additional information from the caregiver regarding the ordered therapy. In another embodiment, information may be solicited from a responsible caregiver (e.g., a pharmacist, nurse, etc.) via a mechanism other than the order entry interface 110 such as, for example, a pharmacy management system, an electronic medical records system, or other appropriate interface.

In any regard, the prompting 308 may include soliciting additional information regarding the therapy order, requesting clarification of conflicting information, or otherwise requesting information needed to prepare a configuration output corresponding to the therapy order. Accordingly, the method 300 may include receiving 310 information from the responsible caregiver. In an embodiment, the receiving 310 of the information may be required prior to generation 316 of the configuration output.

Additionally, the method 300 may also include converting 312 data to a form conforming to the configuration protocol. The converting 312 may include modification of information from the therapy order to a form that corresponds directly to the configuration protocol of the identified patient care device 140. For example, a portion of the therapy information may include information described in units of measure different than the unit of measure designation used in the configuration protocol. Traditionally, an administering caregiver 130 may be required to perform such a conversion manually to correct for the discrepancy between the units of measure of the order and the units of measure used in the configuration protocol of the patient care device 140. However, the configuration output generator 120 may be operable to automatically perform the converting 312 such that data appearing in the resulting configuration output conforms to the form of the configuration protocol.

The method 300 may also include arranging 314 the information in the configuration output to correspond to a data input sequence of the configuration protocol. That is, the configuration protocol may have a sequence of data inputs that are provided in a specific order. The order may have at least a portion of information that is not arranged corresponding to the data input sequence of the configuration protocol. Therefore, in a traditional approach, an administering caregiver 130 may be required to search the order to find the appropriate therapy information from a plurality of potential locations of an order. As may be appreciated this may result in the administering caregiver 130 misreading, entering the therapy information in a sequence that does not correspond to the data input sequence of the configuration protocol, entering therapy information from memory, or otherwise erring, thus resulting in the input of incorrect values. However, in the method 300, as the information in the configuration output may be arranged in correspondence with the data input sequence of the configuration protocol, the administering caregiver 130 may be able to sequentially enter the information for the configuration protocol in a manner that allows the caregiver to simply follow the data input sequence of the configuration output. This may assist the administrating caregiver 130 in accurately and precisely transcribing values from the configuration output to the patient care device 140.

Additionally, the method 300 may include generating 316 the configuration output. The generating 316 may include preparing the configuration output in any appropriate form. In this regard, the generating 316 may include generation of the configuration output on a display (i.e., preparing a soft copy of the configuration order), generating a hard copy of the configuration output, and/or preparing the configuration output as machine-readable data. As described above, the configuration output may be communicated to the patient care device 140. In this regard, the soft copy output of the configuration output may be displayed at the patient care device 140.

In an embodiment, the generating 316 may include printing a label that may be associated with an object used in the administration of the ordered therapy. For example, as will be described in greater detail below, in the context of an IV fluid 252 to be administered using an infusion device 200, the label 262' may be attached to a receptacle 200 for the IV fluid 252. Further still, the generating 316 may include preparing machine-readable data in an appropriate format for distribution to and/or use directly by the patient care device 140. In this regard, the generating 316 may include generation of computer-readable data that may be provided directly to a patient care device 140 for configuration the patient care device 140.

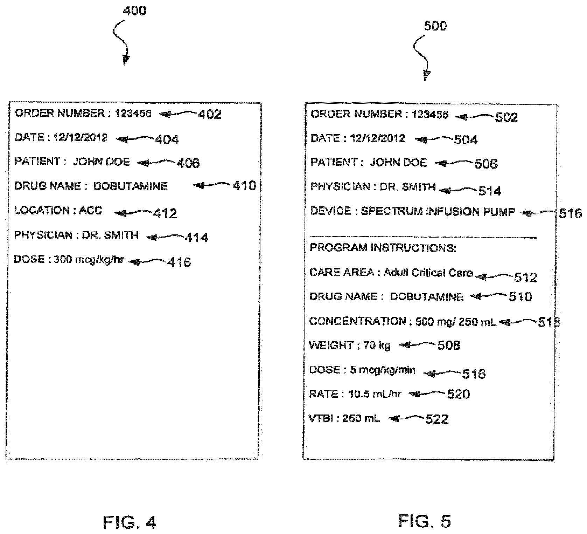

As referenced above, the method 300 of Fig. 3 may be utilized in the context of preparing a configuration output for the administration of an IV fluid 252 to a patient using an infusion device 200. With reference to Figs. 4-7E, one such example is described hereinbelow. For example, in Fig. 4, a dose order 400 corresponding to an ordered dose to be administered to a patient may be received. It may be appreciated that the dose order 400 may be in the form of data stored in memory. Therefore, the display of the dose order 400 in Fig. 4 in human-readable form may be for illustration purposes.

As shown in Fig. 4, the dose order 400 may include a plurality of portions of dose information. While the portions of dose information in Fig. 4 include an order number 402, an order date 404, a patient name 406, a drug name 410, a patient location 412, an ordering physician 414, and a dose amount 416, it may be appreciated that fewer or additional portions of information may be provided with a dose order 400 and that the specific portions shown in Fig. 4 are for explanation purposes and are not intended to be limiting.

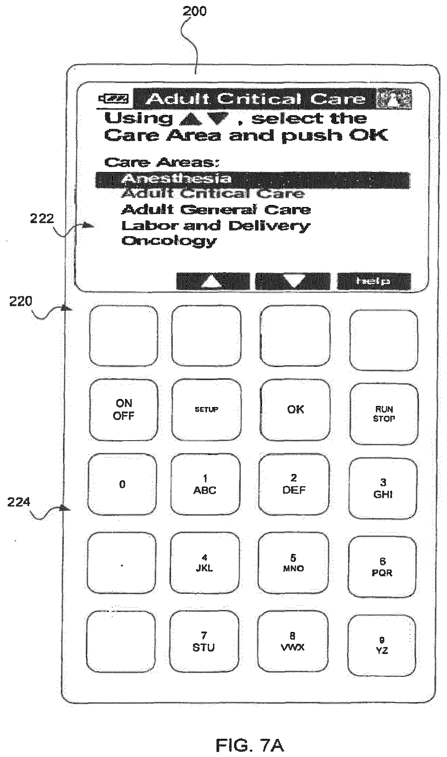

A configuration output generator 120 may receive the dose order 400 and generate the configuration output 500 (e.g., according to method 300). In this regard, it may be determined that the IV fluid 252 corresponding to the dose order 400 is to be administered by an infusion device 200 of the type shown in Figs. 7A-7E. In this regard, Figs. 7A-7E may depict an embodiment of a predetermined configuration protocol for the infusion device 200. As may be appreciated, the predetermined configuration protocol of the infusion device 200 may further have a particular data input sequence in which the various parameters for configuring the infusion device 200 are requested from a user. As such, in Figs. 7A-7E each successive figure may represent a subsequent GUI 220 state in the data input sequence for receiving configuration data in the predetermined configuration protocol for configuring the infusion device 200. In this regard, the configuration output 500 shown in Fig. 5 may be generated based on the dose order 400 and the identification of the infusion device 200 as the patient care device to be used to administer the IV fluid 252 described by the dose order 400.

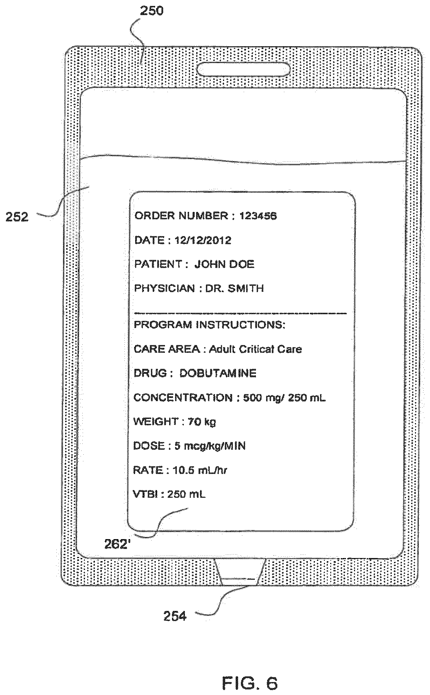

Furthermore, as shown in Fig. 6, the configuration output 500 may comprise a label 262' that is applied to the receptacle 250 for the IV fluid 252 to be administered. In this regard, the IV fluid 252 may be a compounded IV fluid admixture that is prepared by a pharmacy. The pharmacy may also include the configuration output generator 120 such that the configuration output 500 is generated substantially concurrently with the preparation of the IV fluid 252. Thus, once the IV fluid 252 has been prepared and disposed in the receptacle 250, the configuration output 500 may be applied in the form of the label 262' to the receptacle 250 for the IV fluid 252. In this regard, the label 262' may be an adhesive backed label that Is applied to the receptacle 250 prior to the dispensation of the receptacle 250 from the pharmacy. As such, the receptacle 250 including the IV fluid 252 and the configuration output 500 may be collectively delivered to an administering caregiver 130 for subsequent administration to a patient.

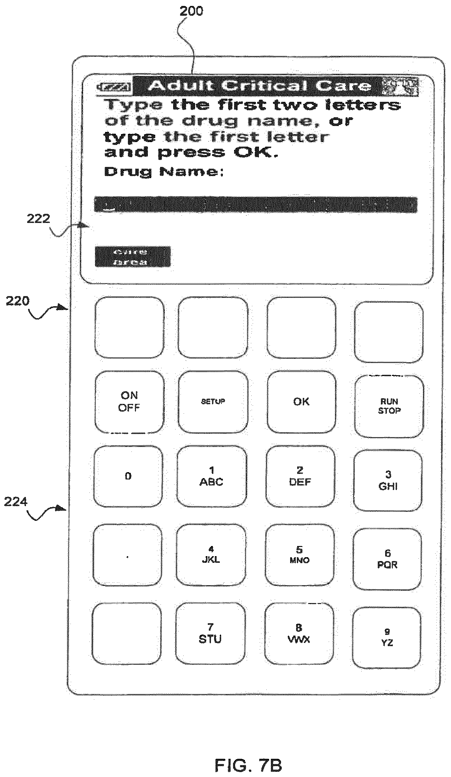

Accordingly, at the initiation of the configuration protocol as shown in Fig. 7A, the configuration protocol may include selection of a care area in which the IV fluid 252 is to be administered (e.g., which may correspond to a patient location in the care facility). As may be appreciated, the dose order 400 included a patient location 412. In this regard, with further reference to Fig. 5, the information associated with the patient location 412 may be modified for inclusion in a corresponding care area data field 512 in the configuration output 500. For example, rather than being designated as a "LOCATION" as per the dose order 400, the configuration output 500 may modify the listing of the patient location information 412 to be designated as a "CARE AREA" in the care area data field 512 that corresponds to the requested input as shown in Fig. 7A. Furthermore, the patient location 412 may be abbreviated as "ACC" in the dose order 400. In the care area data field 512 of the configuration output 500 the value may be converted to "Adult Critical Care" to correspond to the specific input shown in the display 222 of the GUI 220 as shown in Fig. 7A. In this regard, the administering caregiver 130 may use the GUI 220 of the infusion device 200 to select "Adult Critical Care". The selection of the care area may result in the GUI 220 changing to the state shown in Fig. 7B (e.g., advancing the data input sequence of the configuration protocol).

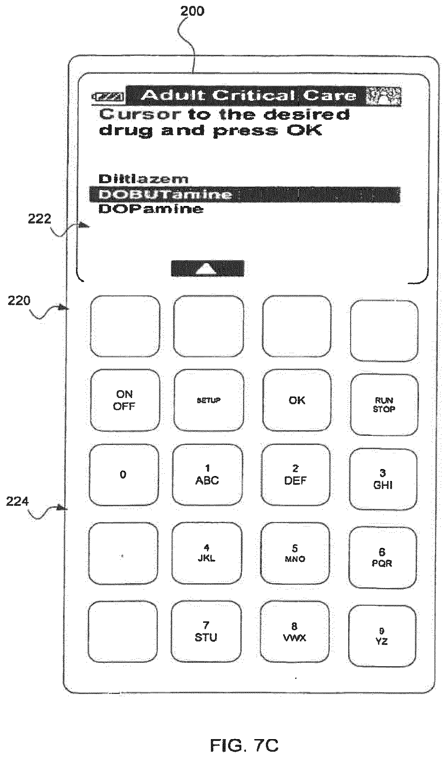

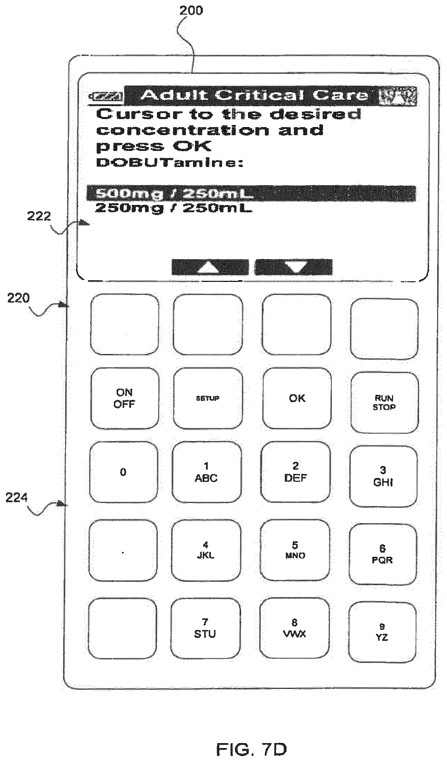

In Fig. 7B, the GUI 220 may prompt the administering caregiver 130 for the name of the drug to be administered. The drug name 412 from the dose order 400 may be included in the configuration output 500. As can be appreciated in Fig. 5, the drug name information 512 may appear directly sequentially after the patient location information 512 such that the arrangement of the care area 512 and the drug name 510 correspond to the data input sequence of configuration protocol for the infusion device 200. In this regard, the administering caregiver 130 may use the input device 224 to enter the drug name as shown in Fig. 7C based upon the drug name 510 provided on the configuration output 500. Upon selection of the drug to be administered, the GUI 220 may change to the state shown in Fig. 7D (e.g., advancing the data input sequence of the configuration protocol).

In Fig. 7D, the concentration of the drug to be administered at the infusion device 200 is to be selected. However, as may be appreciated in Fig. 4, the concentration information for the drug to be administered may not be present in the dose order 400. In this regard, as was discussed in Fig. 3 with respect to method 300, a responsible caregiver may be prompted to provide the information corresponding to concentration information prior to generation of the configuration output based on the configuration output generator 120 determining the parameter is not present in the dose order 400 and included the configuration protocol of the infusion device 200. In this regard, it may be required that a responsible caregiver provide the information prior to the configuration output 500 being generated. In this case, the responsible caregiver may be the ordering physician, a pharmacist, a pharmacist technician, or other responsible caregiver capable of providing the information. For example, such information may be absent as the ordering caregiver may not be required to provide this information or may not be cognizant of the options for the concentrations of the specific drug that are available. For example, in this particular example the concentration of the drug may be determined by other factors such as the available stock in the pharmacy compounding the IV fluid 252, the method used to compound the IV fluid 252, or other factors such as the type of infusion device 220 to be used to administer the IV fluid 252. In another embodiment, the information corresponding to dose concentration 518 in the configuration output may be populated automatically during the compounding of the IV fluid 252 (e.g., a pharmacy work flow management system may be in operative communication with the configuration output generator 120 to automatically provide the information to the configuration output generator 120). In any regard, the drug concentration information 518 may be provided on the configuration output 500 so that the administering caregiver 130 may select the appropriate corresponding selection from the GUI 220 shown in Fig 7D.

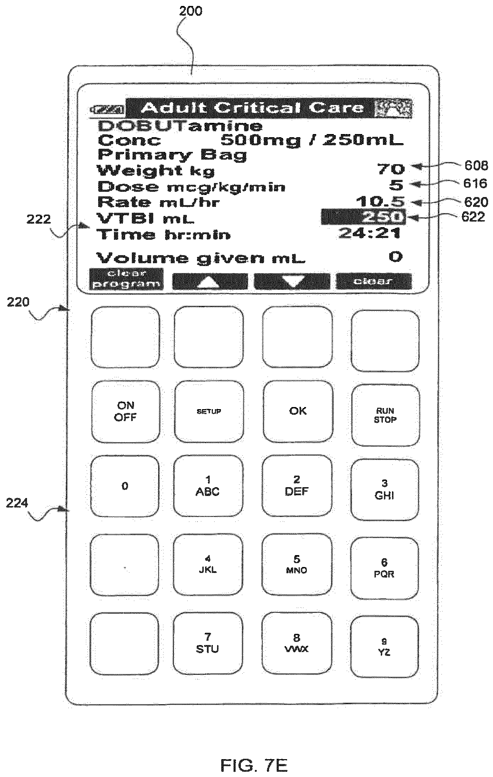

In this regard, the GUI 220 may change to the state shown in Fig. 7E (e.g., advancing the data input sequence of the configuration protocol) where the administering caregiver 130 may be prompted for information in fields corresponding to patient weight 608, dose amount 616, dose administration rate 620, and volume to be infused (VTBI) 622. As may be appreciated, at least a part of the corresponding portions of data (dose amount 616, dose administration rate 620, and VTBI 622) are provided in a corresponding order on the configuration output 500. However, it may be understood that because information corresponding to the patient weight 508 was not provided in the dose order 400, the patient weight 508 may have been received from a responsible caregiver (e.g., a doctor, nurse, or other caregiver) in a manner as described above. Alternatively, the information may be provided by an electronic medical records (EMR) server that includes the information. As such, the configuration output generator 120 may be in operative communication with an EMR server and may be operable to automatically receive at least a portion of the data included in the configuration output 500.

In any regard, as may be appreciated from a collective review of the dose order 400 and the configuration output 500, the arrangement of the portions of the configuration output 500 corresponding to the values to be entered in Fig. 7E may be altered in arrangement from the dose order 400 to the configuration output 500. Additionally, the dose amount information 412 appearing in the dose order 400 appears in units of measure of micrograms per kilogram per hour. However, as may be appreciated in Fig. 7E, the configuration protocol for the infusion device 200 may prompt the administering caregiver 130 for this information in units of measure of micrograms per kilogram per minute. That is, there may be discrepancy in the units of measure used in the dose order 400 and the units of measure required for configuring the infusion device 200. As such, a unit conversion may be performed on the dose amount information 416 to generate the dose amount information 516 of the configuration output.

As may be further appreciated, the rate information 520 and VTBI 522 may not be present in the dose order 400. These values may be provided based on calculations employing other values present in the dose order 400 and/or the configuration output 500 and may be provided in an appropriate conforming form in the configuration output 500. For example, in the depicted embodiment, these values may be derived once the concentration of the drug 518 has been provided or obtained by the configuration output generator 120. It should be noted that the rate information 520 and the VTBI 522 are presented in the appropriate order and in units of measure corresponding to the data input sequence of the configuration protocol of the infusion device 200.

While the invention has been illustrated and described in detail in the drawings and foregoing description, such illustration and description is to be considered as exemplary and not restrictive in character. For example, certain embodiments described hereinabove may be combinable with other described embodiments and/or arranged in other ways (e.g., process elements may be performed in other sequences). Accordingly, it should be understood that only the preferred embodiment and variants thereof have been shown and described and that all changes and modifications that come within the spirit of the invention are desired to be protected.

Further features, aspects and embodiments are provided in the following numbered items:
Item 1. A method for generation of a patient care device-specific configuration output for a therapy order corresponding with a therapy to be administered to a patient, comprising:
   receiving (302) an order that includes at least a portion of a therapy description corresponding to a therapy to be administered to a patient;
   identifying (304) a patient care device to be used to administer the therapy to the patient, wherein the patient care device has at least one predetermined configuration protocol for therapy administration; and
   generating (316) a configuration output for the order, for use in configuring the patient care device for administration of the therapy, based at least in part on at least a portion of each of the corresponding therapy description and the predetermined configuration protocol.
Item 2. The method of Item 1, wherein the therapy to be administered comprises administration of an IV fluid (252) to a patient using an infusion device (200).
Item 3. The method of Item 1 or Item 2, wherein the order comprises a dose order including a dose description corresponding to the IV fluid (252) to be administered.
Item 4. The method of Item 2 or Item 3, wherein the IV fluid (252) comprises at least one of the following:
   a compounded IV admixture;
   a premixed IV solution;
   a customer-prepared non-patient specific fluid; or
   any other type of IV fluid.
Item 5. The method of any one of Items 1-4, wherein at least one of the identifying step (304) and the generating step (316) is at least partially completed in a computer-automated manner.
Item 6. The method of any one of Items 1-5, further comprising: generating a label (262, 262') comprising the configuration output; and affixing the label (262, 262') to a receptacle (250) associated with the therapy.
Item 7. The method of any one of Items 1-6, wherein the predetermined configuration protocol comprises a predetermined data input sequence for input of a plurality of patient care device configuration data.
Item 8. The method of Item 6 or Item 7, wherein the configuration output is presented on the label (262) in accordance with at least a portion the predetermined data input sequence.
Item 9. The method of Item 8, further comprising:
   inputting a corresponding plurality of patient care device configuration data from the configuration output presented on the label (262) to the patient care device (140) in accordance with the predetermined data input sequence.
Item 10. The method of Item 9, wherein the inputting includes entering the corresponding plurality of patient care device configuration data at the patient care device (140) manually by a user.
Item 11. The method of Item 8 or Item 9, wherein operation of the patient care device (140) is at least partially based on the inputting step.
Item 12. The method of any one of Items 9-11, wherein the plurality of patient care device configuration data comprise at least one of the following types of data:
   data indicative of a location of the patient within a patient care facility;
   data indicative of one of a predetermined plurality of dose types;
   data indicative of at least one therapeutically active drug;
   data indicative of dose concentrations;
   data indicative of an administration rate; and
   data indicative of an administration amount.
Item 13. The method of any one of Items 1-12, wherein the identifying step (304) includes:
   identifying one of a plurality of types of patient care devices to be used to administer the therapy to the patient, wherein each one of the plurality of patient care devices has a different predetermined configuration protocol.
Item 14. The method of any one of Items 1-13, wherein the identifying step (304) is completed, based at least in part, upon a location of the patient within a patient care facility.
Item 15. The method of any one of Items 1-14, wherein the generating (316) step is completed, based at least in part, upon a location of the patient within a patient care facility.
Item 16. The method of any one of Items 1-15, further comprising: analyzing the therapy description in relation to at least a portion of the predetermined configuration protocol to determine if the therapy description conforms to the portion of the configuration protocol.
Item 17. The method of Item 16, further comprising:
   providing an output alert at a user interface device when the therapy description is in nonconformance with the portion of the configuration protocol; and
   receiving, in response to the output alert, therapy information corresponding to the order that conforms to the portion of the configuration protocol, wherein the therapy information is used in the generating step (316).
Item 18. The method of Item 17, further comprising:
   requiring receipt of the therapy information that conforms to the portion of the configuration protocol prior to completion of the generating step.
Item 19. The method of Item 18, wherein the portion of the configuration protocol comprises at least one configuration parameter.
Item 20. The method of any one of Items 17-19, wherein the nonconformance of the therapy description comprises at least one missing configuration parameter.
Item 21. The method of Item 19, wherein the nonconformance of the therapy description comprises contradictory information related to the at least one configuration parameter.
Item 22. The method of any one of Items 16-21, further comprising: converting (312) at least a portion of the therapy description from a first form that does not conform with the portion of the configuration protocol to a second form that does conform to the portion of the configuration protocol.
Item 23. The method of Item 22, wherein the converting step includes: performing a unit of measure conversion on at least one portion of the dose description.
Item 24. The method of any one of Items 16-23, wherein the portion of the configuration protocol comprises the entire configuration protocol for the identified patient care device (140).
Item 25. The method any one of Items 1-24, wherein the identifying step (304) is at least partially determined based on at least a portion of the order.
Item 26. The method of Item 25, wherein the identifying step (304) includes:
   assigning the order to a patient care device at least partially based on a portion of the order.
Item 27. The method of Item 25 or Item 26, wherein the portion of the order on which the identifying step (304) is at least partially based comprises at least one of:
   an identity of the therapy; or
   a location of the patient.
Item 28. The method of any one of Items 1-27, wherein the configuration output includes an identification of the patient care device (140) to be used to administer the therapy to the patient.
Item 29. The method of any one of Items 1-28, further comprising: communicating the configuration output to the patient care device (140).
Item 30. The method of Item 29, further comprising:
   displaying the configuration output at the patient care device (140).
Item 31. A system for generation of a patient care device-specific configuration output for use in configuring a patient care device (140) to administer a therapy to a patient, comprising:
   an order entry interface (110) operable to receive a therapy order that includes at least a portion of a therapy description corresponding to a therapy to be administered to a patient; and
   a configuration output generator (120), in operative communication with the order entry interface (110), that is operable to identify a patient care device (140) to be used to administer the therapy to the patient, wherein the patient care device (140) has at least one predetermined configuration protocol for therapy administration; and
   a configuration output that is generated by the configuration output generator (120) and is based at least in part on at least a portion of each of the corresponding therapy description and the predetermined configuration protocol.
Item 32. The system of Item 31, wherein the therapy comprises administration of an IV fluid (252) to a patient using an infusion device (200).
Item 33. The system of Item 32 or Item 33, wherein the therapy order comprises a dose order including a dose description corresponding to the IV fluid (252) to be administered.
Item 34. The system of any one of Items 31-33, wherein the IV fluid (252) comprises at least one of the following:
   a compounded IV admixture;
   a premixed IV solution; and
   a stock IV fluid.
Item 35. The system of any one of Items 31-34, wherein the order entry interface (110) is executed by a processor in operative communication with a memory.
Item 36. The system of Item 35, wherein the memory stores non-transitory computer readable data corresponding to the execution of the order entry interface (110).
Item 37. The system of any one of Items 31-36, wherein the configuration output generator (120) is executed by a processor in operative communication with a memory.
Item 38. The system of Item 37, wherein the memory stores non-transitory computer readable data corresponding to the execution of the configuration output generator (120).
Item 39. The system of any one of Items 31-38, wherein the configuration output comprises a label (262).
Item 40. The system of Item 39, wherein the label (262) is applied to a receptacle (250) for use in administration of the therapy.
Item 41. The system of any one of Items 31- 40, further comprising:
   a patient care device (140) of a corresponding type identified by the configuration output generator (120), the patient care device (140) having a user interface for configuration of the patient care device (140).
Item 42. The system of any one of Items 31-41, wherein the user interface corresponds to the configuration protocol of the patient care device (140).
Item 43. The system of any one of Items 40-42, wherein the label (262) includes one or more portions of configuration data corresponding to at least a portion of the configuration protocol.
Item 44. The system of any one of Items 40-43, wherein the one or more portions of configuration data comprises at least one of the following types of data:
   data indicative of a location of the patient within a patient care facility;
   data indicative of one of a predetermined plurality of dose types;
   data indicative of at least one therapeutically active drug;
   data indicative of dose concentrations;
   data indicative of an administration rate; and
   data indicative of an administration amount.
Item 45. The system of any one of Items 31-44, wherein the configuration protocol comprises a predetermined data input sequence.
Item 46. The system of Item 45, wherein the label includes a corresponding plurality of configuration data presented on the label (262) in accordance with the predetermined data input sequence.
Item 47. The system of Item 45 or Item 46, wherein the user interface is operable to receive the corresponding plurality of configuration data in the predetermined data input sequence.
Item 48. The system of Item 46 or Item 47, wherein the corresponding plurality of configuration data is manually entered to the patient care device (140) by a user.
Item 49. The system of any one of Items 31-48, wherein the patient care device (140) is a configurable infusion pump.
Item 50. The system of any one of Items 40-49, wherein the receptacle (250) includes an IV fluid (252), wherein the receptacle (250) is in operative communication with an administration set (256), and wherein the administration set (256) is operatively engaged by the configurable infusion pump for controlled delivery of the IV fluid (252) to a patient.
Item 51. The system of Item 49 or Item 50, wherein the IV fluid (252) is administered to the patient at least partially based on the corresponding plurality of configuration data input at the configurable infusion pump.

## Claims

1. A method for generation of a patient care device-specific configuration output, comprising:
by an order entry interface, receiving (302) an order that includes at least a portion of a therapy description corresponding to a therapy to be administered to a patient;
by a configuration output generator, identifying (304) a patient care device to be used to administer the therapy to the patient, wherein the patient care device has at least one predetermined configuration protocol for therapy administration;
by the configuration output generator, analyzing (306) the therapy description in relation to the predetermined configuration protocol to determine if the therapy description conforms to the predetermined configuration protocol; and
by the configuration output generator, generating (316) a configuration output for the order comprising patient care device configuration data for use in manual configuring of the patient care device for administration of the therapy, the configuration output being based at least in part on at least a portion of each of the corresponding therapy description and the predetermined configuration protocol.

2. The method of Claim 1, wherein the therapy to be administered comprises administration of an IV fluid (252) to a patient using an infusion device (200),
wherein the IV fluid (252) may comprise at least one of the following:
a compounded IV admixture;
a premixed IV solution;
a customer-prepared non-patient specific fluid; or
any other type of IV fluid.

3. The method of Claim 2, wherein the order comprises a dose order including a dose description corresponding to the IV fluid (252) to be administered.

4. The method of Claim 1 or 2, wherein at least one of the identifying step (304) and the generating step (316) is at least partially completed in a computer-automated manner.

5. The method of any one of Claims 1 or 2, wherein the predetermined configuration protocol comprises a predetermined data input sequence for input of a plurality of patient care device configuration data.

6. The method of Claim 5, wherein the configuration output is presented on a label (262) in accordance with at least a portion the predetermined data input sequence.

7. The method of Claim 6, further comprising:
by a user interface of the patient care device, inputting a corresponding plurality of patient care device configuration data from the configuration output presented on the label (262) to the patient care device (140) in accordance with the predetermined data input sequence.

8. The method of Claim 7, wherein the inputting includes entering the corresponding plurality of patient care device configuration data at the patient care device (140) manually by the user interface;
wherein operation of the patient care device (140) may be at least partially based on the inputting step;
wherein the plurality of patient care device configuration data may comprise at least one of the following types of data:
data indicative of a location of the patient within a patient care facility;
data indicative of one of a predetermined plurality of dose types;
data indicative of at least one therapeutically active drug;
data indicative of dose concentrations;
data indicative of an administration rate; and data indicative of an administration amount.

9. The method of Claim 1 or 2, wherein the identifying step (304) includes:
by the configuration output generator, identifying one of a plurality of types of patient care devices to be used to administer the therapy to the patient, wherein each one of the plurality of patient care devices has a different predetermined configuration protocol.

10. The method of Claims 1 or 2, further comprising:
by the configuration output generator, analyzing the therapy description in relation to at least a portion of the predetermined configuration protocol to determine if the therapy description conforms to the portion of the configuration protocol.

11. The method of Claim 10, further comprising:
by the user interface, providing an output alert when the therapy description is in nonconformance with the portion of the configuration protocol; and
by the configuration output generator, receiving, in response to the output alert, therapy information corresponding to the order that conforms to the portion of the configuration protocol, wherein the therapy information is used in the generating step (316);
wherein the method may further comprise:
by the configuration output generator, requiring receipt of the therapy information that conforms to the portion of the configuration protocol prior to completion of the generating step.

12. The method of Claim 10, further comprising:
by the configuration output generator, converting (312) at least a portion of the therapy description from a first form that does not conform with the portion of the configuration protocol to a second form that does conform to the portion of the configuration protocol.

13. A system for generation of a patient care device-specific configuration output for use by a user when transcribing information from the configuration output to a patient-care device to configure a patient care device (140) to administer a therapy to a patient, comprising:
an order entry interface (110) operable to receive a therapy order that includes at least a portion of a therapy description corresponding to a therapy to be administered to a patient;
a configuration output generator (120), in operative communication with the order entry interface (110), that is operable to:
identify a patient care device (140) to be used to administer the therapy to the patient, wherein the patient care device (140) has at least one predetermined configuration protocol for therapy administration,
analyze the therapy description in relation to the predetermined configuration protocol to determine if the therapy description conforms to the predetermined configuration protocol, and
generate a configuration output for the order comprising patient care device configuration data for use in manual configuring of the patient care device for administration of the therapy, the configuration output being based at least in part on at least a portion of each of the corresponding therapy description and the predetermined configuration protocol.

14. The system of Claim 13, further comprising:
a patient care device (140) of a corresponding type identified by the configuration output generator (120), the patient care device (140) having a user interface for configuration of the patient care device (140) in response to manual entry of the patient care device configuration data.

15. The system of Claim 13 or 14, wherein a label (262) includes one or more portions of configuration data corresponding to at least a portion of the configuration protocol;
wherein the patient care device (140) may be a configurable infusion pump.
